# EUROPEAN PATENT SPECIFICATION

(11) **EP 1 414 470 B1**
(45) Date of publication and mention of the grant of the patent: **28.03.2007**
(21) Application number: 02753916.2
(22) Date of filing: 06.08.2002
(51) Int. Cl.: A61K 31/6615, A61K 31/661, A61P 31/00, A23L 1/302, A23L 1/303, A61K 31/122, A61K 31/355, A61K 31/07, A61K 31/375

(54) **MICRONUTRIENT PHOSPHATES AS DIETARY AND HEALTH SUPPLEMENTS**
MIKRONÄHRSTOFF-PHOSPHATE ALS NAHRUNGSERGÄNZUNGSMITTEL UND MITTEL ZUR FÖRDERUNG DER GESUNDHEIT
PHOSPHATES MICRONUTRIANTS UTILISES COMME SUPPLEMENTS SANITAIRES ET DIETETIQUES

(30) Priority: 06.08.2001 AU PR684801
(43) Date of publication of application: 06.05.2004
(73) Proprietor: Vital Health Sciences Pty Ltd., Melbourne, VIC 3000 (AU)
(72) Inventor: WEST, Simon, Michael, Williamstown, VIC 3016 (AU); KANNAR, David, Belgrave South, VIC 3160 (AU)
(74) Representative: Guerre, Dominique
(86) International application number: PCT/AU2002/001081
(87) International publication number: WO 2003/013550

(56) References cited:
- EP-A- 0 430 045
- EP-A- 0 684 043
- EP-A- 0 845 216
- EP-B- 0 565 007
- WO-A-02/26238
- WO-A-93/15731
- US-A- 4 684 520
- US-A- 5 114 957
- US-A- 5 603 949
- US-A- 5 643 597
- US-A- 5 776 915
- FRACALOSSI D.M. ET AL: 'Oscars, Astronotus ocellatus, have a dietary requirement for vitamin C' J. OF NUTRITION vol. 128, no. 10, October 1998, pages 1745 - 1751, XP002976887
- BLOM J.H. ET AL: 'Reproductive success of female rainbow trout (Oncorhynchus mykiss) in response to graded dietary ascorbyl monophosphate levels' BIOLOGY OF REPRODUCTION vol. 52, no. 5, May 1995, pages 1073 - 1080, XP002976888

## Description

The invention relates to dietary or health supplements for improved delivery of micronutrient compounds. More particularly, the invention relates to dietary or health supplements for improved delivery of micronutrient compounds which are electron transfer agents.

### Background of the invention

In this specification, where a document, act or item of knowledge is referred to or discussed, this reference or discussion is not an admission that the document, act or item of knowledge or any combination thereof was at the priority date:
(a) part of common general knowledge; or
(b) known to be relevant to an attempt to solve any problem with which this specification is concerned.

Whilst the following discussion mainly concems ubiquinol and tocopherol, it is to be understood that this is merely illustrative and that the invention is not limited to these electron transfer agents.

Coenzyme Q10 (CoQ₁₀) or ubiquinone is lipophilic because it has ten repeating isoprene units. It is an endogenous essential cellular constituent that is present in every cell of the body and serves as a coenzyme for several key steps in the production of energy within the cell. CoQ₁₀ is regarded as being of fundamental importance as it is reported to pay an important physiological role in the mitochondrial transport of electrons and production of energy within mitochondria of each cell.

CoQ₁₀ is made available to the body through endogenous biosynthesis and dietary intake. Folkers suggests that a CoQ₁₀ deficiency leading to evidence of a clinically significant disease state may occur because of:
- insufficient dietary CoQ₁₀
- impairment in CoQ₁₀ biosynthesis,
- excessive utilization of CoQ₁₀ by the body,
or any combination of the three. As CoQ₁₀ is essential to the optimal function of all cell types, it is not surprising to find a seemingly diverse number of disease states which respond favorably to CoQ₁₀ supplementation. All metabolically active tissues may therefore be highly sensitive to CoQ₁₀ deficiency. It is reasonable to assume that optimal nutrition (which may in future include optimal levels of CoQ₁₀) is generally beneficial in many disease states.

As CoQ₁₀ improves the cell respiratory chain and stabilizes mitochondrial membranes, it has a potential role in some cardiac insufficiency diseases associated with aging. CoQ₁₀ is known to be highly concentrated in heart muscle cells due to the high energy requirements of this cell type. Whether primary, secondary or both, this deficiency of CoQ₁₀ may be a major treatable factor in the otherwise inexorable progression of heart failure. Other nutrients reported to be of potential use in treatment of cardiovascular diseases including vitamin E, inosine, cytochrome C, or treatment of hyperhomocysteinemia with oral folate, betaine and/or pyridoxine therapy could also be considered. These nutrients may assist in treating a range of diseases associated with age and stimulate endogenous CoQ₁₀ production.

### Dietary supplement

CoQ₁₀ shows a high variability in its absorption, with some subjects attaining good blood levels of CoQ₁₀ on 100 mg per day while others require two or three times this amount to attain the same blood level. All CoQ₁₀ presently available in the United States is manufactured in Japan and is distributed by a number of companies who place the CoQ₁₀ either in pressed tablets, powder-filled capsules, or oil-based soft gel capsules- CoQ₁₀ is fat-soluble and absorption should be improved when administered with dietary fat. Published data on the dosage of CoQ₁₀ relates almost exclusively to the treatment of disease states. There is no information on the use of CoQ₁₀ for prevention of illness. This is an extremely important question which, to date, does not have an answer.

Absorption is reported to take place through:
(a) the formation of micelles with biliary salts in a similar fashion to vitamin A; or
(b) after direct adhesion of CoQ₁₀ to the intestinal membrane, by passive transport (that is strongly inhibited by its high molecular weight); and/or
(c) lipoprotein transport. However, when administered orally, absorption is highly variable and dependent upon formulation parameters.

CoQ₁₀ is a poorly soluble quinone. Intestinal absorption of CoQ₁₀ can be improved with effective formulation. There is evidence that fat soluble nutrients are better absorbed from aqueous or emulsified vehicles than from oily preparations.

| **Formulation** | **Daily Dose** | **Baseline CoQ10 Blood level** | **Peak CoQ10 Blood level** | **% Change (@ C_{Max}** |
|---|---|---|---|---|
| Powder (particle size <125µm) | 100 mg capsule | 16525±1598 µg l⁻¹-h | 21197±2046 µg l⁻¹-h | 28% |
| Powder | 60 mg tablet | 0.45 µg/ml | 0.98 µg/ml | 118% |
| Powder | 100 mg capsule | 630±165 nmol/L | 736±156 nmol/L | 17% |
| Granular | 90 mg | 1.00±0.31 µmol/l | 1.81±0.82 µmol/l change | 168% |
| Oil suspension | 120 mg soft gel | 0.40±0.11 µg/ml | 1.26±0.50 µg/ml | 215% |
| Oil base | 90 mg capsule | 1.07±0.34 µmol/l | 1.90±0.97 µmol/l change | 178% |
| Oil base | 90 mg soft gel | 0.98±0.29 mg/l | 2-03±0-58 mg/l | 107% |
| Oil suspension (Glyceryl monoleate) | 100 mg soft gel | 16525±1598 µg l⁻¹h | 24941±3528 µg l⁻¹h | 51% |
| Lipid Microsphere (Soy oil+egg phospholipids) | 60 mg soft gel | 0.70 µg/ml | 2.62 µg/ml | 274% |
| Oil emulsion (MCT+surfactant) | 100 mg soft gel | 605±121 nmol/L | 1534+384 nmol/L | 253% |
| Oil emulsion (MCT+surfactant+Vit E) | 120 mg soft gel | 0.38±0.11 µg/ml | 2.80±0.80 µg/ml | 637% |
| Oil emulsion (Soy lecithin+Gelucine) | 100 mg capsule | 16525±1598 µg l⁻¹-h | 52857±1949 µg l⁻¹-h | 220% |
| Plasma Vit E & Vit C | | - | - | No change |

Despite these increases in bioavailability, it is important to note that absolute absorption still remains less than optimal. It is suggested that the increase in bioavailability noted in the above table is due to the oil emulsion surfactant system increasing solubility and dissolution rate, or the ability of surfactants to penetrate and disrupt biological membranes increasing permeability of the drug load. This assumes that uptake through the intestinal membrane is a passive process.

A leading American CoQ₁₀ (Q-Gel) soft gelatine capsule uses a proprietary formula containing CoQ₁₀ in a blend of sorbitan monooleate, polysorbate 80, medium chain triglycerides (MCT's), propylene glycol, d-alpha tocopherol, PVP (Plasdone) and annato seed extract which has been reported to increase CoQ₁₀ bioavailability compared to other commercial formulations in US patent no 6,056,971.

Other methods of solubilising CoQ₁₀ have been reported including hydrogenated castor oil (HCO-60) and ethanol-water (1:5 by vol) and other lipoidal drug delivery systems incorpcrating stable sub micron range particles in lecithin as discussed in US patent no 5,989,583. Lipid microspheres may enhance the absorption of CoQ₁₀.

Other parameters important to CoQ₁₀ absorption include:
(a) awareness of the micelle size as a function of bioavailability,
(b) HLB value of surfactants as a function of bioavailability, as high HLB numbers may improve bioavailability (Pozzi et al. 1991. Weis et al. 1994), and
(c) CoQ10 granule size.

The intestinal absorption of lipid-soluble drugs can be markedly influenced by the ora dosage form as well as the formulation factors. Many commercial vitamin preparation s are formulated as compressed tablets, hardshell gelatin capsules or soft gelatin capsules which contain a complex matrix of excipients, fillers and other adjuvants. Compounds formulated in soft gelatin capsules representing liquid fills tend to be better absorbed :han hard gelatin capsules, which encapsulate a dry powder blend, however little attention has historically been given to bioavailability of dietary supplements.

In a recent study conducted by Walhqvist, the bioavailability of CoQ₁₀ from two different preparations was compared in order to ascertain if the emulsified preparation had higher bioavailability of CoQ₁₀ than a powdered preparation. Two different gelatin capsules containing 50 mg of CoQ₁₀ were used in this study. The first preparation was crystalline CoQ_{10.} with dicalcium phosphate as a filler and magnesium stearate as an excipient, filled in a hard gelatin capsule. The other contained CoQ₁₀ as a complex micelle in an emulsion encapsulated into a soft gelatin capsule. The conclusion of the study was that the emulsified soft gel capsule had a higher bioavailability than the powder in hard gel formulation used in this study. The presence of surfactants in the soft gel formulation would contribute to the enhanced solubilisation and release of CoQ₁₀.

Despite all this research into delivery of CoQ₁₀, the absorption levels which have been achieved are not yet optimal.

### Foods with additional micronutrients

Appreciation of dietary CoQ₁₀ intake is important when considering formulation of dietary supplements and functional foods for a number of reasons. The chemistry of the compound is also important and can indicate preferred forms utilized by the body. Vitamin B6 for example, can be found in the free form (pyridoxine), a glycoside (pyridoxamine) and supplied in dietary supplements as a hydrochloride salt (pyridoxine hydrochloride). In foods, the vitamin primarily exists as a phosphate (pyridoxal 5-phosphate). Bioavailability varies depending upon the type of food and method of preparation but typically the phosphate is better ingested.

Finished product formulations should be representative of the original food source to be certain that other compounds accompanying CoQ₁₀ originally present in the food are present. These dietary compounds are important to consider as they can dramatically after bioavailability of the finished product. So where possible consideration should be given to what compounds are also present. For example foods rich in CoQ₁₀ are typically fatty eg: oily fish and soy oil. It is therefore not surprising then that bioavailability of CoQ₁₀ is reported to improve when formulated with a lipid vehicle.

Consideration of food sources rich in CoQ₁₀ will help identify normal dietary intake levels. In peer reviewed literature there is some variance in opinion on what constitutes an adequate: or effective dose of CoQ₁₀ and some thought that dietary intake could unifonnly be low.

Food preparation is also important to consider and can assist with knowing how these methods affect absorption- For example, the effect of cooking is a 14-32% destruction of CoQ₁₀ by frying, and no detectable destruction by boiling. This suggests that CoQ₁₀ is likely to be heat stable, may be utilized in hot beverages and is likely to be successfully concentrated by moderate heat extraction.

Regular food intakes are important to consider and indicate that in normal individuals a low intake is adequate or that bioavailability is optimal because naturally co-administered compounds present in the food improve absorption.

Reduced CoQ₁₀ (ubiquinol) delivered in supplements has been reported to increase circulating levels of reduced CoQ₁₀. How the molecule is changed into an oxidised form is becoming clearer- Analysis of the actual form of ubiquinol in foods has not been undertaken, nor is it clear what form is preferred by the body. However, it is thought that to act effectively as an electron transfer agent CoQ₁₀ must remain in a reduced form.

There is thus a need for an improved delivery system for micronutrient compounds such as reduced CoQ₁₀ and other important dietary or health supplements.

### Vitamin E

Vitamin E is a potent electron transfer agent capable of protecting polyunsaturated fatty acids (PUFA) within phospholipids of biological membranes and plasma lipoproteins. Vitamin E also stabilizes membranes, modulates protein kinase C activity and positively influences immune response. Although supplementation is popular, only higher dietary consumption is reliably associated with lower risk of coronary heart disease in both men and women on a cross cultural basis.

When provided as a supplement, vitamin E is provided as tocopherol. When delivered as an isolated nutrient, vitamin E is poorly absorbed due to its lipid solubility and chemically unstable due to primary oxidation of the phenolic group. To improve delivery, vitamin E is esterified and presented as simple substituted esters - either succinate or acetate derivatives. While this pro-drug strategy is primarily undertaken to prevent oxidation of the phenolic group, improve lymphatic transport, and enhance stability, increase in tissue tocopherol may take many weeks to achieve.

Although dietary supplementation with vitamin E esters - particularly the natural form - RRR- stereoisomer, may increase the content of α-tocopherol in blood plasma and erythrocytes, bioavailability is still significantly less than optimal with blood levels being subject to wide inter-patient variability and clinical efficacy disappointing.

Luminal events in gastrointestinal lipid digestion have been well studied and a micellar hypothesis of fat absorption established. A number of attempts have therefore been made to enhance α-tocopherol acetate lymphatic transport via lipid formulation approaches. Despite improvements, food can still have a significant impact increasing the extent of α-tocopheryl ester absorption after oral administration, indicating that factors other than dispersion, digestion and solubilisation may be responsible for intestinal uptake of vitamin E. Other lipophilic drugs and nutrients are also subject to poor and variable absorption properties following oral administration including vitamin A, indicating that current self emulsifying drug delivery formulation approaches as well as other lipid-based formulations may be of limited value in increasing bioavialability of poorly soluble lipid compounds.

Being fundamentally important to cellular viability, vitamin E must be transported efficiently and mobilised on demand to act as an electron transfer agent and not reach too high a concentration to become pro-oxidant. This delicate biological balance must start with effective transport across the small intestine mucosa, yet this process is currently not well understood.

Tocopheryl phosphate (TP) is a more water-soluble analogue of tocopherol and proposed to have higher bioavailability than tocopheryl acetate (TA) most likely because of nore efficient intestinal uptake. As a water-soluble analogue TP is easier to formulate in functional foods, and dietary supplements but many enzymes in the gastrointestinal tract have phosphorylase activity and reduce the amount of TP delivered to the small intestine. TP also forms acid insoluble complexes that may reduce the amount of product available for transport across the intestinal wall.

There is a need for a delivery system which effectively provides improved delivery of a portion of the daily allowance of micronutrient compounds such as vitamin E and CoQ₁₀.

EP-A-0 430 045 relates to pharmaceutical compositions as inhibitors of Maillard's reaction comprising in admixture with a pharmaceutically acceptable carrier a ascorbic acid tocopheryl phosphate diester.

US-A-5 776 915 discloses novel retinoid phosphocholines exhibiting anti-tumor, anti-psoriatic and anti-inflammatory activities.

US-A-4 684 520 realtes to novel pharmaceutical compositions containing ubiquinone, particularly Coenzyme Q10, and mixtures of phospholipids having organic or vegetal origin, together with pharmaceutically acceptable excipients.

The article of Fracalossi DM et al. (J. of Nutrition, vol. 128, no. 10, October 1998, pp 1745-1751) teaches that vitamin C is an essential nutrient for an Amazonian ornamental fish. Vitamin C is supplemented as L-ascorbyl-2-polyphosphate, a mixture of phosphate esters of ascorbate, which is more stable to oxidation than ascorbic acid.

The article of Blom JH et al. (Biology of Reproduction, vol. 52, no. 5, May 1995, pp 1073-1080) relates to the reproductive success of female rainbow trout in response to graded dietary ascorbyl monophosphate levels.

EP-B-0 565 007 relates to a bath agent containing L-ascorbic acid-tocopherolphosp late diester, having the effect of removing and preventing the skin itch and preventing the skin aging.

EP-A-0 684 043 discloses a therapeutic composition for corneal impairment containing an ascorbyl tocopheryl phosphate compound or a pharmacologically acceptable salt thereof EP-A-0 845 216 relates to a tocopheryl phosphate, a salt thereof and a composition containing the tocopheryl phosphate or a salt thereof as a source of vitamin E for administration to animals.

US-A-5 114 957 relates to methods for inhibiting viral and retroviral replication via the administration of compositions containing vitamin E, tocopherol, or a tocopherol derivative and, more particularly, compositions containing α-tocopherol or a pharmaceutically effective prodrug thereof.

US-A-5 603 949 and US-A-5 643 597 disclose the use of a tocopherol phosphate ether than alpha-tocopherol phosphate for the preparation of cosmetic or pharmaceutical compositions.

WO 93 15731 A relates to phosphate-substituted vitamin E for protecting cells from effects of aging and from chemically-induced cell injury and for stimulating cell repair.

### Summary of the invention

It has been discovered that the provision of a dietary or health supplement comprising micronutrient compounds is markedly improved by use of the micronutrient in the form of phosphate derivatives.

According to the invention, the micronutrient is the reaction product of (a) one or more phosphate derivatives of ubiquinol, ascorbic acid, tocotrienol, tocopherol and mixtures thereof and (b) one or more complexing agents selected from the group consisting of amphoteric surfactants, cationic surfactants, amino acids having nitrogen functional groups and proteins rich in these amino acids.

The term "phosphate derivatives" comprises compounds covalently bound by means of an oxygen to the phosphorus atom of a phosphate group. The oxygen atom is typically derived from a hydroxyl group on the micronutrient. The phosphate derivative may exist in the form of a free phosphate acid, a saft thereof, a phosphate ester having two molecules of micronutrient, a mixed phosphate ester having two different micronutrients, a phosphatidyl compound wherein the free phosphate oxygen forms a bond with an alkyl or substituted alkyl group and complexes with amphoteric surfactants, cationic surfactants, amino acids having nitrogen functional groups and proteins rich in these amino acids

Preferably, the phosphate mixtures consist of one mono-micronutrient phosphate derivative and one di-micronutrient phosphate derivative wherein the amount of mono-micronutrient phosphate derivative is no less than equimolar to the amount of di-micronutrient phosphate derivative as disclosed in international patent application no pCT/AU01/01475. For example, a mixture containing 70% ubiquinyl phosphate and 26% di-ubiquinyl phosphate.

Phosphorylation may be accomplished by any suitable method. Preferably, the hydroxyl group-containing micronutrient is phosphorylated using P₄O₁₀ according to the method in international patent application no PCT/AU00/00452. Excess diphosphate derivatives may be hydrolyzed using methods known to those skilled in the art.

In some situations, it may be necessary to use a phosphate derivative such as a phosphatide where additional properties such as increased water solubility are preferred. Phosphatidyl derivatives are amino alkyl derivatives of organic phosphates. These derivatives may be prepared from amines having a structure of R₁R₂N(CH₂)ₙOH wherein n is an integer between 1 and 6 and R₁ and R₂ may be either H or short alkyl chains with 3 or less carbons. R₁ and R₃ may be the same or different. The phosphatidyl derivatives are prepared by displacing the hydroxyl prolon of the micronutrient with a phosphate entity that is then reacted with an amine, such as ethanolamine or N,N' dimethylethanolamine, to generate the phosphatidyl derivative of the micronutrient. One method of prepare tion of the phosphatidyl derivatives uses a basic solvent such as pyridine or triethylamine with phosphorous oxychloride to prepare the intermediate which is then reacted with the hydroxy group of the amine to produce the corresponding phosphatidyl derivative, such as P cholyl P ubiquinyl dihydrogen phosphate.

According to the invention there is provided a dietary or health supplement comprising an effective amount of a micronutrient selected from the group consisting of complexes of phosphate derivatives of ubiquinol, ascorbic acid, retinol, tocotrienol, tocopherol and mixtures thereof delivered with an acceptable carrier.

The term complexes of phosphate derivatives of a micronutrient" refers to the reaction product of (a) one or more phosphate derivatives of ubiquinol, ascorbic acid, tocotrienol, retinol, tocopherol and mixtures thereof and (b) one or more complexing agents selected from the group consisting of amphoteric surfactants cationic surfactants, amino acids having nitrogen functional groups and proteins rich in these amino acids as disclosed in international patent application no PCT/AU01/01476.

The preferred complexing agents are selected from the group consisting of arginine, lysine and tertiary substituted amines, such as those according to the following formula:

NR¹R²R³

wherein R¹ is chosen from the group comprising straight or branched chain mixed alkyl radicals from C6 to C22 and carbonyl derivatives thereof;
R² and R³ are chosen independently from the group comprising H, CH₂COOX,
CH₂CHOHCH₂SO₃X, CH₂CHOHCH₂OPO₃X, CH₂CH₂COOX, CH₂COOX,
CH₂CH₂CHOHCH₂SO₃X or CH₂CH₂CHOHCH₂OPO₃X and X is H, Na, K or alkanolamine provided R² and R³ are not both H; and
wherein when R¹ is RCO then R² may be CH₃ and R³ may be (CH₂CH₂)N(C₂H₄OH)-H₂CHOP0₃ or R² and R³ together may be N(CH₂)₂N(C₂H₄OH)CH₂COO-

Examples of such complexes of phosphate derivatives of a micronutrient are formed by the reaction of any combination of A) tocopheryl phosphate, retinyl phosphate, ascorbyl phosphate, tocotrienyl phosphate, ubiquinyl phosphate or mixtures thereof with B) arginine, lysine or lauryliminodipropionic acid where complexation occurs between the alkaline nitrogen center and the phosphoric acid ester to form a stable complex.

The term "effective amount" refers to a portion or multiple of the daily allowance of each micronutrient which provides a bioactive effect on the subject. It is recognized that lipophilic substances are not readily excreted or metabolised so it is unusual to supply a large multiple of the recommended daily allowance (***RDA***) in a food source. It is recommended that typically any non medical use of dietary supplements should contain less than the recommended than the RDA and typically a third of the RDA. But it is recognised that for chronic medical uses, it is desirable to supply large multiples of the RDA for rapid increase in recovery.

The effective amount of the one or more phosphate derivatives of a micronutrient may be a concentration in the range of from 10 ppm to 10,000 ppm (w/w) of the dietary or health supplement Preferably, the one or more phosphate derivatives of a micronutrient is added at a concentration of 50 ppm to 1,000 ppm (w/w) in accordance with the need to supply the recommended daily allowance or a small multiple thereof.

The term "dietary or health supplement" as used in this description refers to all forms of supplying micronutrient compounds. For example, tablets, powders, chewable tablets, capsules, nasal delivery solutions, food additives, oral suspensions, children's formulations, enteral feeds, parenteral nutrition (for example, intravenous needs), nutraceuticals and functional foods. Preferably, the dietary or health supplements in a form selected from but not limited to the group consisting of capsule, tablet, powder and foods such as cookie, biscuit, breakfast cereal, sports drink and sports food bar. A person skilled in the art would know the acceptable carriers and other excipients which could be used in the invention. Typically, if the one or more phosphate derivatives of a micronutrient, such as ubiquinyl phosphate, is lipophilic then there is a lipidic carrier used such as medium chain triglycerides.

Use of an effective amount of a micronutrient together with an acceptable carrier in the manufacture of a dietary or health supplement for supplementing a subject's intake of a daily allowance of the micronutrient, wherein the micronutrient is selected from the group consisting of complexes of phosphate derivatives of ubiquinol, ascorbic acid, retinol, tocotrienol, tocopherol and mixtures, thereof.

A dietary or health supplement when used for supplementing a subject's intake of a micronutrient, the dietary or health supplement comprising an effective amount of one or more complexes of phosphate derivatives of ubiquinol, ascorbic acid, retinol, tocotrienol, tocopherol and mixtures thereof and an acceptable carrier.

### Examples

The invention will now be further explained and illustrated by reference of the accompanying non-limiting examples.

### Example 1 (this example is for information purpose only)

In this example, ubiquinyl phosphate was prepared. 100g ubiquinol was heated to 100 °C and 33g of P₄O₁₀ was added. The mixture was stirred for 3 hours and 500 ml water was then introduced slowly into the mixture. The temperature of the reaction was maintained jus; below boiling point for a further 1 hour. Removal of water yielded ubiquinyl phosphate, and inorganic phosphates. The inorganic phosphates were removed by further washes with hot water. The remaining amorphc us material was then mixed with 100 L of virgin grade canola oil containing at least 1 to 5% lecithin. The final mixture of ubiquinyl phosphate in canola oil at a concentration of 1 mg/ml was incorporated into supplements such as capsules and functional foods.

### Example 2

The preparation method for tocopheryl phosphate arginine complex is as follows:

The molar ratio of the compounds arginine, NaOH and mixture of tocopheryl phosphate/ditocopheryl phosphate (free acid form) is nominally 1:1:1, but a slight excess of argin ne and NaOH was employed. A saturated solution of NaOH (60% w/w) was added to the dry arginine and stirred at 70°C for 20 minutes. Water (30 ml for every 150 g of TP/T₂P to be used) was added to facilitate better mixing. The tocophenyl phosphate/ditocopheryl phosphate mixture was added to the solution and stirred vigorously with a high-shear mixer at 70°C for 1 hour.

### Example 3

In this example, the stability of the tocopheryl phosphate arginine complex (***TP***) in a beverage was investigated.

Tocopheryl phosphate arginine complex (equivalent α-tocopherol content of 50 mg/500ml) was added to commercially available Musashi drinks (Musashi, Australia) a blue variety and an orange variety.

A 2% (w/v) tocopheryl phosphate arginine complex stock solution was prepared in water and filter-sterilised using a Millipore Millex-GP 25 mm, diameter 0.22 µm filter and sterile hypodermic syringe. Four ml was added to each 500 ml bottle using sterile conditions.

Three treatment groups were used for each drink: 4°C, 37°C and room temperature (RT). The drinks were monitored for bacterial growth and contamination on a weekly basis, both visually, and by plating a sample onto LB agar plates, which were grown for 48 hours at 37°C.

The results showed that there was no bacterial or fungal contamination in any of the samples within each treatment group.

### pH results:

| **Treatment group** | **4°C** | **37°C** | **RT** |
|---|---|---|---|
| Blue group (no TP) | 4.27 | 4.28 | 4.11 |
| Blue group (TP) | 4.34 | 4.31 | 4.16 |
| Orange group (no TP) | 3.40 | 3.27 | 3.26 |
| Orange group (TP) | 3.42 | 3.29 | 3.28 |

### Turbidity Measurements

| **NTU results** | **4°C** | **37°C** | **RT** |
|---|---|---|---|
| Blue group (no TP) | 0.08 | 0.23 | 0.06 |
| Blue group (TP) | 12.54 | 28.55 | 24.05 |
| Orange group (no TP) | 218.0 | 190.0 | 206.0 |
| Orange group (TP) | 232.0 | 220.0 | 224.0 |

Drinks are made acidic to ensure microbial stability. There is detectable turbidation at low pH. However, at the pH required for biological stability, the amount of degradation of the tocopheryl phosphate arginine complex was negligible. This form of vitamin E supplementation is useful for such drinks.

## Claims

1. A dietary or health supplement comprising an effective amount of a micronutrient selected from the group consisting of complexes of phosphate derivatives of ubiquinol, ascorbic acid, retinol, tocotrienol, tocopherol and mixtures thereof delivered with an acceptable carrier,
wherein the micronutrient is the reaction product of (a) one or more phosphate derivatives of ubiquinol, ascorbic acid, tocotrienol, retinol, tocopherol and mixtures thereof and (b) one or more complexing agents selected from the group consisting of amphoteric surfactants, cationic surfactants, amino acids having nitrogen functional groups and proteins rich in these amino acids.

2. A dietary or health supplement according to claim 1 wherein the micronutrient is selected from the group consisting of the reaction product of
(a) a micronutrient selected from the group consisting of tocopheryl phosphate, retinyl phosphate, ascorbyl phosphate, tocotrienyl phosphate, ubiquinyl phosphate or mixtures thereof; and
(b) a complexing agent selected from the group consisting of arginine, lysine or lauryliminodipropionic acid.

3. A dietary or health supplement according to claim 2 wherein the micronutrient is ubiquinyl phosphate arginine complex.

4. A dietary or health supplement according to claim 2 wherein the micronutrient is tocopheryl phosphate arginine complex.

5. A dietary or health supplement according to claim 1 wherein the complexes of phosphate derivatives of a micronutrient is added at a concentration of from 10 ppm to 10,000 ppm % (w/w).

6. A dietary or health supplement according to claim 5 wherein the micronutrient is added at a concentration of from 50 ppm to 1,000 ppm % (w/w).

7. A dietary or health supplement according to claim 1 selected from the group consisting of capsule, tablet, powder, food additives, cookie, biscuit, breakfast cereal, sports drink and sports food bar.

8. A dietary or health supplement according to claim 7 being a capsule.

9. A dietary or health supplement according to claim 7 being a sports drink.

10. A dietary or health supplement according to claim 7 being a cookie containing ubiquinyl phosphate.

11. A dietary or health supplement according to any one of claims 8 to 10 containing tocopheryl phosphate arginine complex.

12. Use of an effective amount of a micronutrient selected from the group consisting of complexes of phosphate derivatives of ubiquinol, ascorbic acid, retinol, tocotrienol, tocopherol and mixtures thereof together with an acceptable carrier in the manufacture of a dietary or health supplement for supplementing a subject's intake of a daily allowance of the micronutrient,
wherein the micronutrient is the reaction product of (a) one or more phosphate derivatives of ubiquinol, ascorbic acid, tocotrienol, retinol, tocopherol and mixtures thereof and (b) one or more complexing agents selected from the group consisting of amphoteric surfactants, cationic surfactants, amino acids having nitrogen functional groups and proteins rich in these amino acids.

13. Use according to claim 12, wherein the micronutrient comprises tocopheryl phosphate arginine complexes.

## Patentansprüche

1. Nahrungs- oder Gesundheitsergänxungsmittel mit einer effektiven Menge an einem Mikronährstoff, der ausgewählt ist aus der Gruppe bestehend aus Komplexen von Phosphatderivaten von Ubiquinol, Ascorbinsäure, Retinol, Tocotrienol, Tocopherol und Mischungen davon, die mit einem akzeptablen Träger abgegeben werden,
wobei der Mikronährstoff das Reaktionsprodukt ist aus (a) einem oder mehreren Phosphatderivaten von Ubiquinol, Ascorbinsäure, Tocotrienol, Retinol, Tocopherol und Mischungen davon, und (b) einem oder mehreren komplexbildenden Stoffen, die ausgewählt sind aus der Gruppe bestehend aus amphoteren oberflächenaktiven Stoffen, kationischen oberflächenaktiven Stoffen, Aminosäuren mit Stickstoff-funktionalen Gruppen, sowie Proteinen, die reich an diesen Aminosäuren sind.

2. Nahrungs- oder Gesundheitsergänzungsmittel nach Anspruch 1, bei welchem der Mikronährstoff ausgewählt ist aus der Gruppe bestehend aus dem Reaktionsprodukt aus
(a) einem Mikronährstoff, der ausgewählt ist aus der Gruppe bestehend aus Tocopherylphosphat, Retinylphosphat, Ascorbylphosphat, Tocotrienylphosphat, Ubiquinylphosphat oder Mischungen daraus; und
(b) einem komplexbildenden Stoff, der ausgewählt ist aus der Gruppe bestehend aus Arginin, Lysin oder Luuryliniinodipropionsäure.

3. Nahrungs- oder Gesundheitsergänzungsmittcl nach Anspruch 2, bei welchem der Mikronährstoff ein Ubiquinylphosphat-Arginin-Komplex ist.

4. Nahrungs- oder Gesundheitscrgänzungsmittel nach Anspruch 2, bei welchem der Mikronährstoff ein Tocopherylphosphat-Arginin-Komplex ist.

5. Nahrungs- oder Gesundheibergänzungsmittel nach Anspruch 1, bei welchem die Komplexe aus den Phosphatderivaten eines Mikronährstoffes mit einer Konzentration von 10 ppm bis 10.000 ppm % (w/w) hinzugefügt werden.

6. Nahrungs- oder Gesundheitsergänzungsmittel nach Anspruch 5, bei welchem der Mikronährstoff mit einer Konzentration von 50 ppm bis 1.000 ppm % (w/w) hinzugefügt wird.

7. Nahrungs- oder Gesundheitsergänzungsmittel nach Anspruch 1, das ausgewählt ist aus der Gruppe bestehend aus einer Kapsel, Tablette, Pulver, Nahrungsergänzungsstoffen, Keksen, Brötchen, Frühstückszerealien, Sportgetränken und Sportnahrungsriegeln.

8. Nahrungs- oder Gesundheitsergänzungsmittel nach Anspruch 7, das eine Kapsel ist.

9. Nahrungs- oder Gesundheitsergänzungsmittel nach Anspruch 7, das ein Sportgetränk ist.

10. Nahrungs- oder Gesundheitsergänzungsmittel nach Anspruch 7, das ein Keks ist, der Ubiquinylphosphat enthält.

11. Nahrungs- oder Gesundheitsergänzungsmittel nach einem der Ansprüche 8 bis 10, das einen Tocopherylphosphat-Arginin-Komplex enthält.

12. Verwendung einer effektiven Menge eines Mikronährstoffes, der ausgewählt ist aus der Gruppe bestehend aus Komplexen von Phosphatderivaten von Ubiquinol, Ascorbinsäure, Retinol, Tocotrienol, Tocopherol und Mischungen davon, zusammen mit einem akzeptablen Träger für die Herstellung eines Nahrungs- oder Gesundheitsergänzungsmittel zur Ergänzung der Aufnahme einer täglich empfohlenen Menge des Mikronährstoffes für eine Person,
wobei der Mikronährstoff das Reaktionsprodukt ist aus (a) einem oder mehreren Phosphatderivaten von Ubiquinol, Ascorbinsäure, Tocotrienol, Retinol, Tocopherol und Mischungen davon, und (b) einem oder mehreren komplexbildenden Stoffen, die ausgewählt sind aus der Gruppe bestehend aus amphoteren oberflächenaktiven Stoffen, kationischen oberflächenaktiven Stoffen, Aminosäuren mit Stickstoff-funktionalen Gruppen und Proteinen, die reich an diesen Aminosäuren sind.

13. Verwendung nach Anspruch 12, bei welcher der Mikronährstoff Tocopherylphosphat-Arginin-Komplexe aufweist.

## Revendications

1. Supplément alimentaire ou diététique comprenant une quantité efficace d'un micronutriment choisi dans le groupe constitué par les complexes de dérivés phosphate de l'ubiquinol, de l'acide ascorbique, du rétinol, du tocotriénol, du tocophérol et de mélanges de ceux-ci, délivrée avec un support acceptable,
le micronutriment étant le produit de réaction de (a) un ou plusieurs dérivés phosphate de l'ubiquinol, de l'acide ascorbique, du tocotriénol, du rétinol, du tocophérol et de mélanges de ceux-ci et de (b) un ou plusieurs agents complexants choisis dans le groupe constitué par les tensioactifs amphotères, les tensioactifs cationiques, les acides aminés ayant des groupes fonctionnels azote et les protéines riches en ces acides aminés.

2. Supplément alimentaire ou diététique selon la revendication 1, **caractérisé en ce que** le micronutriment est choisi dans le groupe constitué par le produit de réaction de
(a) un micronutriment choisi dans le groupe constitué par le phosphate de tocophéryle, le phosphate de rétinyle, le phosphate d'ascorbyle, le phosphate de tocotriényle, le phosphate d'ubiquinyle ou des mélanges de ceux-ci ; et
(b) un agent complexant choisi dans le groupe constitué par l'arginine, la lysine ou l'acide lauryliminodipropionique.

3. Supplément alimentaire ou diététique selon la revendication 2, **caractérisé en ce que** le micronutriment est un complexe phosphate d'ubiquinyle-arginine.

4. Supplément alimentaire ou diététique selon la revendication 2, **caractérisé en ce que** le micronutriment est un complexe phosphate de tocophéryle-arginine.

5. Supplément alimentaire ou diététique selon la revendication 1, **caractérisé en ce que** les complexes de dérivés phosphate d'un micronutriment sont ajoutés à une concentration comprise entre 10 ppm et 10 000 ppm %(p/p).

6. Supplément alimentaire ou diététique selon la revendication 5, **caractérisé en ce que** le micronutriment est ajouté à une concentration comprise entre 50 ppm et 1 000 ppm %(p/p).

7. Supplément alimentaire ou diététique selon la revendication 1, choisi dans le groupe constitué par une capsule, un comprimé, une poudre, des additifs alimentaires, un cookie, un biscuit, des céréales de petit-déjeuner, une boisson pour sportif et une barre nutritive pour sportif.

8. Supplément alimentaire ou diététique selon la revendication 7, étant une capsule.

9. Supplément alimentaire ou diététique selon la revendication 7, étant une boisson pour sportif.

10. Supplément alimentaire ou diététique selon la revendication 7, étant un cookie contenant du phosphate d'ubiquinyle.

11. Supplément alimentaire ou diététique selon l'une quelconque des revendications 8 à 10, contenant un complexe phosphate de tocophéryle-arginine.

12. Utilisation d'une quantité efficace d'un micronutriment choisi dans le groupe constitué par les complexes de dérivés phosphate de l'ubiquinol, de l'acide ascorbique, du rétinol, du tocotriénol, du tocophérol et des mélanges de ceux-ci, conjointement avec un support acceptable, dans la fabrication d'un supplément alimentaire ou diététique destiné à supplémenter la prise d'un apport quotidien en micronutriment d'un individu,
le micronutriment étant le produit de réaction de (a) un ou plusieurs dérivés phosphate de l'ubiquinol, de l'acide ascorbique, du tocotriénol, du rétinol, du tocophérol et de mélanges de ceux-ci et de (b) un ou plusieurs agents complexants choisis dans le groupe constitué par les tensioactifs amphotères, les tensioactifs cationiques, les acides aminés ayant des groupes fonctionnels azote et les protéines riches en ces acides aminés.

13. Utilisation selon la revendication 12, **caractérisée en ce que** le micronutriment comprend des complexes phosphate de tocophéryle-arginine.
